# FASCICULE DE BREVET EUROPEEN

(11) **EP 0 438 072 B1**
(45) Date de publication et mention de la délivrance du brevet: **23.03.1994**
(21) Numéro de dépôt: 91100195.6
(22) Date de dépôt: 08.01.1991
(51) Int. Cl.: A47J 37/04, F24C 15/16

(54) **Four électrique combiné micro-ondes et résistance chauffante**
Elektrischer Ofen, kombiniert mit Mikrowellen und Heizelement
Electrical oven combined with micro-waves and heating resistance

(30) Priorité: 10.01.1990 FR 9000235
(43) Date de publication de la demande: 24.07.1991
(73) Titulaire: MOULINEX, F-93170 Bagnolet (FR)
(72) Inventeur: Chartrain, Pierre, Moulinex, F-14 123 Cormelles-Le-Royal (FR); Gensbittel, Olivier Henri Jean-Louis, Moulinex, F-14 123 Cormelles-Le-Royal (FR)
(74) Mandataire: May, Hans Ulrich, Dr.

(56) Documents cités:
- EP-A- 0 332 505
- DE-A- 2 226 498
- DE-A- 2 422 633
- FR-A- 2 105 460
- FR-A- 2 342 048
- US-A- 3 100 435
- US-A- 3 199 438
- US-A- 3 691 937
- US-A- 4 455 467

## Description

L'invention se rapporte à un four électrique combiné destiné à cuire des aliments au moyen d'une résistance chauffante associée à un chauffage par micro-ondes.

L'invention concerne, plus particulièrement, un four du type comprenant, dans une cavité limitée par des parois et une porte, une lèchefrite équipée d'un moyen de support recevant une broche et pouvant occuper deux positions, soit une position de cuisson pour laquelle la lèchefrite est à l'intérieur de la cavité et la broche est amenée dans l'axe d'un entraîneur rotatif monté sur l'une des parois, soit une position de retrait pour laquelle la lèchefrite est au moins partiellement à l'extérieur de la cavité et la broche est libérée de l'entraîneur.

Un appareil de ce type est décrit dans la demande de brevet FR-2 342 048.

Dans les fours combinés micro-ondes et résistance chauffante connus, il est difficile d'accoupler parfaitement dans l'axe la broche avec l'entraîneur. L'utilisateur a alors tendance à entrer la broche en force, ce qui risque d'endommager l'entraîneur qui, dans un four à micro-ondes, est en céramique et donc est une pièce fragile.

L'invention a pour but de remédier notamment à cet inconvénient et de réaliser un four combiné micro-ondes/gril dans lequel l'accouplement de la broche avec l'entraîneur, d'une part, soit simple, aisé et sans risque de brûlures pour l'utilisateur et, d'autre part, ne risque pas d'endommager l'entraîneur.

Selon l'invention, la lèchefrite et les parois de la cavité comportent des moyens de coopération qui comportent des surfaces de glissement mutuelles agencées de manière que, préalablement à la position de cuisson, les surfaces exercent les unes sur les autres une force dirigée vers l'intérieur du four tendant à positionner la lèchefrite automatiquement en sa position de cuisson.

Ainsi, grâce à cette réalisation, on obtient, en déplaçant seulement la lèchefrite, l'introduction de la broche et son centrage automatique pour réaliser l'accouplement avec l'entraîneur. En effet, une fois que la lèchefrite est amenée en une position préalable à la position de cuisson, le poids de la pièce à rôtir suffit seul à faire avancer la lèchefrite jusqu'au fond de la cavité, par simple réaction des surfaces de glissements les unes sur les autres. L'utilisateur n'est plus gêné par l'obscurité pouvant régner dans la cavité ni par la pièce à rôtir qui masque l'entraîneur et ainsi ne risque pas d'endommager l'entraîneur. Lors du retrait, on obtient également le désengagement de la broche de l'entraîneur, et ceci sans toucher à la broche. L'utilisateur, n'ayant pas besoin de mettre ses mains dans la cavité pour installer ou démonter la broche, ne risque plus de se brûler . Un autre avantage de l'invention est qu'elle est applicable aussi bien aux broches disposées transversalement qu'aux broches disposées longitudinalement par rapport aux parois latérales de la cavité.

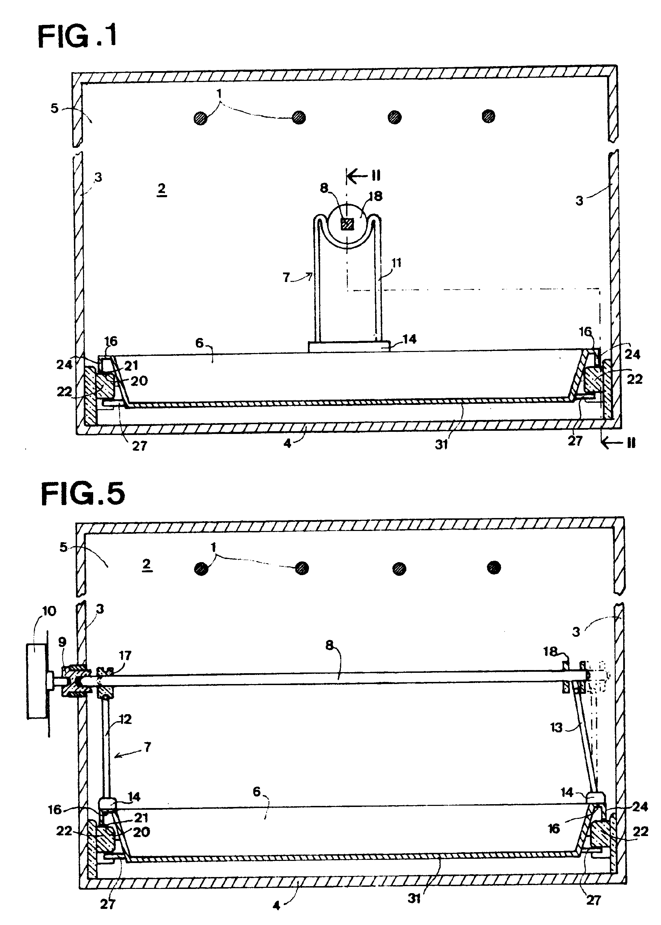

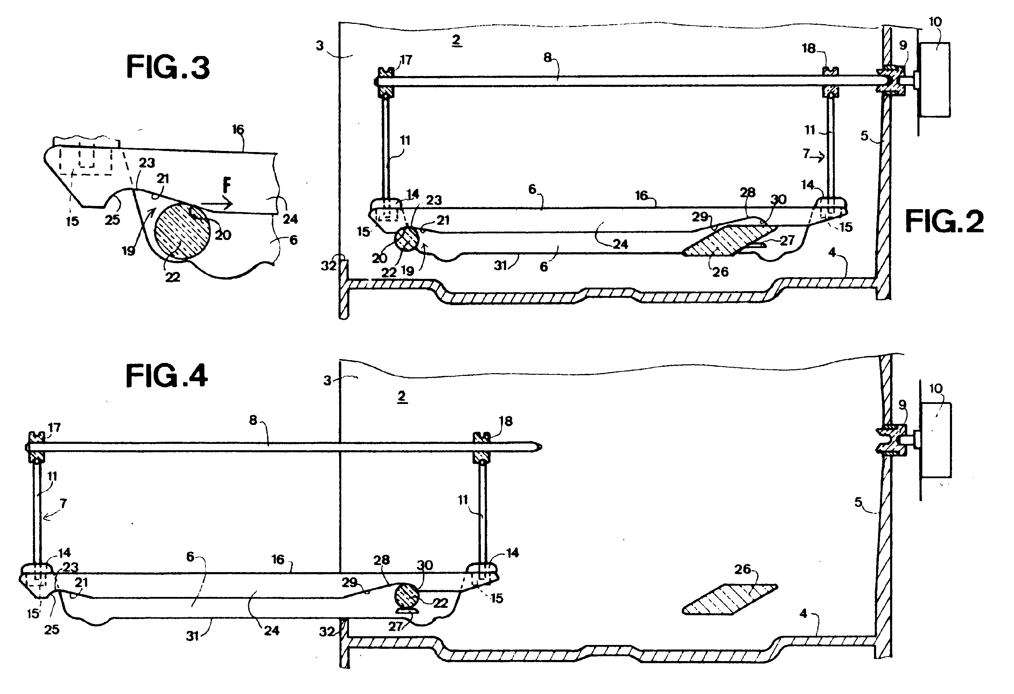

Les caractéristiques et avantages de l'invention ressortiront d'ailleurs de la description qui va suivre, à titre d'exemple, en référence aux dessins annexés dans lesquels
la figure 1 est une vue en coupe transversale d'un four selon l'invention, dans lequel l'entraîneur est disposé sur la paroi de fond de la cavité; la figure 2 est une vue en coupe longitudinale partielle selon la ligne II.II de la figure 1 illustrant la lèchefrite en position de cuisson; la figure 3 est une vue agrandie des moyens de coopération illustrant la lèchefrite dans une position préalable à la position de cuisson; la figure 4 est une vue analogue à la figure 2 illustrant la lèchefrite en position de retrait; la figure 5 est une vue en coupe transversale d'un four selon l'invention, dans lequel l'entraîneur est disposé sur une paroi latérale de la cavité.

Le four électrique combiné tel que représenté sur les figures 1 à 5 est destiné à cuire des aliments au moyen d'une résistance chauffante 1 associée à un chauffage par micro-ondes au moyen d'un magnétron (non représenté). Il comprend, dans une cavité 2 limitée par des parois 3,4,5 et une porte (non représentée), une lèchefrite 6 en métal équipée d'un moyen de support 7 recevant une broche métallique 8 et pouvant occuper deux positions, soit une position de cuisson (figures 1, 2 et 5) pour laquelle la lèchefrite 6 est à l'intérieur de la cavité 2 et la broche 8 est amenée dans l'axe d'un entraîneur rotatif 9 en céramique entraîné en rotation par un moteur 10 et monté sur l'une des parois 3,5, soit une position de retrait (figure 4) pour laquelle la lèchefrite 6 est au moins partiellement à l'extérieur de la cavité 2 et la broche 8 est libérée de l'entraîneur 9.

Les moyens de support 7 de la broche 8 sont constitués par deux supports opposés 11,12,13, réalisés en fil métallique, qui sont isolés chacun, d'une part, de la lèchefrite 6, par une base isolante 14 en céramique montée de façon amovible dans des ouvertures 15 pratiquées dans le bord latéral 16 de la lèchefrite 6 et, d'autre part, de la broche 8, par une bague isolante 17,18 en céramique qui est traversée par la broche 8.

Selon l'invention, la lèchefrite 6 et les parois 3 de la cavité 2 comportent des moyens de coopération 19 qui comportent des surfaces de glissement mutuelles 20,21 portées, d'une part, par des butées 22 agencées sur les parois latérales 3 de la cavité à légère distance de la sole 4 et, d'autre part, par des décrochements 23 pratiqués respectivement dans des bords latéraux correspondants 16 de la lèchefrite 6, de manière que, préalablement à la position de cuisson, les surfaces 20,21 exercent les unes sur les autres une force F (figure 3) dirigée vers le fond 5 de la cavité 2 tendant à positionner la lèchefrite 6 automatiquement en sa position de cuisson (figure 2).

Cette force F est créée par la réaction produite sur lesdites surfaces 20,21 par le poids de la pièce à rôtir et de la lèchefrite elle-même. Cette force F fait donc avancer la lèchefrite 6 automatiquement de sa position préalable (figure 3) à sa positon de cuisson (figure 2).

Les butées 22 sont constituées par deux plots cylindriques situés en regard l'un de l'autre constituant chacun une surface de glissement 20 tandis que, les bords latéraux 16 de la lèchefrite 6 présentant des rebords verticaux 24 dirigés vers le bas, les décrochements 23 sont pratiqués dans lesdits rebords 24 et présentent chacun une partie arrondie 25 servant de cran d'arrêt et une partie qui est inclinée de haut en bas dans le sens d'introduction de la lèchefrite 6 dans la cavité 2 et qui constitue l'une 21 des surfaces de glissement.

Deux autres plots 26 en forme de losange sont agencés sur les parois latérales 3 au fond de la cavité pour assurer la bonne stabilité de la lèchefrite 6 en position de cuisson et la mise à niveau de la broche 8 dans l'axe de l'entraîneur 9.

La lèchefrite 6 comporte en outre des pattes 27 disposées sous des encoches 28 pratiquées dans les rebords verticaux 24 de la lèchefrite 6. Les encoches 28 présentent chacune, d'une part, une arète 29 inclinée de haut en bas dans le sens de retrait de la lèchefrite 6, de manière à faciliter le glissement desdites encoches 28 sur les butées 22 et, d'autre part, une partie arrondie 30 de manière que, en position de retrait, chaque butée 22 se trouve coincée entre une patte 27 et une encoche 28, les encoches 28 ayant une profondeur par rapport à la sole 4 telle que, en position de retrait, le fond 31 de la lèchefrite 6 repose sur le bord d'entrée 32 de la cavité 2, de manière à limiter le retrait et le basculement de la lèchefrite 6.

L'utilisation de la lèchefrite 6 selon l'invention est donc particulièrement simple .

La lèchefrite occupant au départ sa position de retrait (figure 4), l'utilisateur, pour la mettre en place à l'intérieur de la cavité 2, la pousse vers le fond 5. Les arètes inclinées 29 des encoches 28 puis les rebords verticaux 24 de la lèchefrite 6 glissent sur les butées 22. Quand la lèchefrite arrive en position dite préalable (figure 3), les parties inclinées 21 des décrochement 23 arrivent en contact avec les butées 22 et font glisser automatiquement la lèchefrite 6 vers le fond de la cavité 2. En même temps, les rebords 24 de la lèchefrite prennent appui sur les plots en losange 26. La lèchefrite 6 vient donc automatiquement occuper sa position de cuisson (figure 2) et la broche 8 est bien dans l'axe de l'entraîneur 9.

Pour retirer la lèchefrite 6, l'utilisateur tire horizontalement sur la lèchefrite jusqu'à la position de retrait (figure 4). Les butées 22 viennent se coincer respectivement entre une patte 27 et une encoche 28 et le fond 31 de la lèchefrite vient en appui sur le bord d'entrée 32. Cette position empêche le basculement de la lèchefrite équipée de la broche et permet à l'utilisateur d'arroser la pièce rôtie ou de contrôler sa cuisson.

Quand l'utilisateur désire ôter entièrement la lèchefrite 6, par exemple pour la nettoyer, il doit repousser légèrement vers l'intérieur la lèchefrite de façon à dégager les butées 22 des pattes 27 puis soulever la lèchefrite 6 au dessus des butées 22.

Dans le mode de réalisation de l'invention représenté sur les figures 1,2 et 4, l'entraîneur 9 est disposé sur la paroi de fond 5 et la broche 8 est disposée longitudinalement à la cavité 2. Les deux supports 11 sont fixes et la broche est maintenue en translation par une bague 17 solidaire de la broche 8 et une bague 18 solidaire d'un support 11, de manière que la broche 8 soit accouplée automatiquement avec l'entraîneur 9 lorsque la lèchefrite 6 est amenée en position de cuisson. Ainsi, lorsque la lèchefrite 6 avance jusqu'à sa position de cuisson, l'extrémité de la broche 8 pénétre automatiquement dans l'entraîneur 9.

Dans un autre mode de réalisation de l'invention représenté sur la figure 5, l'entraîneur 9 est disposé sur une paroi latérale 3 et la broche 8 est disposée transversalement à la cavité 2. Un des supports 12 est équipé d'une bague 17 et est monté fixe sur la lèchefrite 6, tandis que l'autre support 13 est monté mobile et peut basculer vers l'entraîneur 9, de manière que, lorsque la lèchefrite 6 occupe sa position de cuisson, la broche 8 étant solidaire en translation de l'autre bague 18, le basculement dudit support 13 entraîne le coulissement de la broche 8 dans la bague 17 et ainsi son accouplement avec l'entraîneur 9.

Ainsi, lorsque la lèchefrite 6 est en sa position de cuisson, la broche 8 est dans l'axe de l'entraîneur 9 et il suffit de faire basculer le support mobile 13 pour que, par translation, la broche 8 s'accouple avec l'entraîneur 9.

Pendant toute l'opération de cuisson et la rotation de l'entraîneur 9, la force F maintient l'accouplement correct de la broche 8 avec l'entraîneur 9, en empêchant le recul de la lèchefrite 6.

## Revendications

1. Four électrique combiné destiné à cuire des aliments au moyen d'une résistance chauffante (1) associée à un chauffage par micro-ondes et comprenant, dans une cavité (2) limitée par des parois (3,4,5) et une porte, une lèchefrite (6) équipée d'un moyen de support (7) recevant une broche (8) et pouvant occuper deux positions, soit une position de cuisson pour laquelle la lèchefrite (6) est à l'intérieur de la cavité (2) et la broche (8) est amenée dans l'axe d'un entraîneur rotatif (9) monté sur l'une des parois (3,5), soit une position de retrait pour laquelle la lèchefrite (6) est au moins partiellement à l'extérieur de la cavité (2) et la broche (8) est libérée de l'entraîneur (9),
**caractérisé en ce que** la lèchefrite (6) et les parois (3) de la cavité (2) comportent des moyens de coopération (19) qui comportent des surfaces de glissement mutuelles (20,21) agencées de manière que, préalablement à la position de cuisson, les surfaces (20,21) exercent les unes sur les autres une force F dirigée vers le fond (5) de la cavité (2) tendant à positionner la lèchefrite (6) automatiquement en sa position de cuisson.

2. Four électrique combiné selon la revendication 1,
**caractérisé en ce que** les surfaces de glissement (20,21) sont portées, d'une part, par des butées (22) agencées sur les parois latérales (3) de la cavité à légère distance de la sole (4) et, d'autre part, par des décrochements (23) pratiqués respectivement dans des bords latéraux correspondants (16) de la lèchefrite (6).

3. Four électrique combiné selon la revendication 2,
**caractérisé en ce que** les butées (22) sont constituées par deux plots cylindriques situés en regard l'un de l'autre tandis que, les bords latéraux (16) de la lèchefrite présentant des rebords verticaux (24) dirigés vers le bas, les décrochements (23) sont pratiqués dans lesdits rebords (24) et présentent chacun une partie qui est inclinée de haut en bas dans le sens d'introduction de la lèchefrite (6) dans la cavité (2) et qui constitue l'une (21) des surfaces de glissement.

4. Four électrique combiné selon la revendication 3,
**caractérisé en ce que** la lèchefrite (6) comporte en outre des pattes (27) disposées sous des encoches (28) pratiquées dans les rebords verticaux (24) de la lèchefrite de manière que, en position de retrait, chaque butée (22) se trouve coincée entre une patte (27) et une encoche (28), les encoches (28) ayant une profondeur par rapport à la sole (4) telle que, en position de retrait, le fond (31) de la lèchefrite (6) repose sur le bord d'entrée (32) de la cavité (2), de manière à limiter le retrait et le basculement de la lèchefrite (6).

5. Four électrique combiné selon l'une quelconque des revendications précédentes,
**caractérisé en ce que** les moyens de support (7) de la broche (8) sont constitués par deux supports opposés (11,12,13) qui sont isolés chacun, d'une part, de la lèchefrite (6), par une base isolante (14) montée dans des ouvertures (15) pratiquées dans le bord (16) de la lèchefrite et, d'autre part, de la broche (8), par une bague isolante (17,18) qui est traversée par la broche (8).

6. Four électrique combiné selon la revendication 5,
**caractérisé en ce que**, l'entraîneur (9) étant disposé sur la paroi de fond (5) et la broche (8) étant disposée longitudinalement à la cavité (2), les deux supports (11) sont fixes, de manière que la broche (8) soit accouplée automatiquement avec l'entraîneur (9) lorsque la lèchefrite (6) est amenée en position de cuisson.

7. Four électrique combiné selon la revendication 5
**caractérisé en ce que**, l'entraîneur (9) étant disposé sur une paroi latérale (3) et la broche (8) étant disposée transversalement à la cavité (2), un des supports (12) est équipé d'une bague (17) et est monté fixe sur la lèchefrite (6), tandis que l'autre support (13) est monté mobile et peut basculer vers l'entraîneur (9), de manière que, lorsque la lèchefrite (6) occupe sa position de cuisson, la broche (8) étant solidaire en translation de l'autre bague (18), le basculement dudit support (13) entraîne l'accouplement de la broche (8) avec l'entraîneur (9).

8. Four électrique combiné selon l'une quelconque des revendications 5 à 7,
**caractérisé en ce que** la lèchefrite (6) étant métallique, les butées (22), les bases (14) et les bagues isolantes (17,18) et l'entraîneur (9) sont réalisés dans des matériaux diélectriques du type céramique, tandis que la broche (8) et les supports (11,12,13) sont métalliques.

## Patentansprüche

1. Kombinierter elektrischer Ofen zum Garen von Lebensmitteln mittels eines Heizelements (1) kombinert mit einer Mikrowellenheizung, der in einem durch Wände (3, 4, 5) und eine Tür begrenzten Ofenraum (2) eine Tropfschale (6) aufweist, die mit einer Stützvorrichtung (7) zur Aufnahme eines Spießes (8) ausgerüstet ist und zwei Stellungen einnehmen kann, entweder eine Grillstellung, in der sich die Tropfschale (6) im Inneren des Ofenraums (2) befindet und der Spieß (8) in die Achse eines Drehantriebs (9) gebracht ist, der an einer der Wände (3, 5) montiert ist, oder eine zurückgezogene Stellung, in welcher sich die Tropfschale mindestens teilweise außerhalb des Ofenraums (2) befindet, und der Spieß (8) vom Drehantrieb (9) frei ist, **dadurch gekennzeichnet,** daß die Tropfschale (6) und die Wände (3) des Ofenraums (2) Kooperationsvorrichtungen (19) aufweisen, welche aneinander gleitende Flächen (20, 21) aufweisen, die so ausgebildet sind, daß vor Erreichen der Grillstellung die Gleitflächen (20, 21) aufeinander eine zur Rückwand (5) des Ofenraums (2) gerichtete Kraft (F) ausüben, welche bestrebt ist, die Tropfschale (6) automatisch in ihre Brat- oder Grillstellung zu bringen.

2. Kombinierter elektrischer Ofen nach Anspruch 1, **dadurch ge****kennzeichnet,** daß die Gleitflächen (20, 21) einerseits von Anschlägen (22), die an den Seitenwänden (3) des Ofenraums in geringem Abstand vom Boden (4) angeordnet sind, und andererseits von Ausnehmungen (23) gebildet sind, die jeweils in den entsprechenden Seitenkanten (16) der Tropfschale (6) ausgebildet sind.

3. Kombinierter elektrischer Ofen nach Anspruch 2, **dadurch ge****kennzeichnet,** daß die Anschläge (22) aus zwei einander gegenüber angeordneten zylindrischen Klötzen bestehen, während die Seitenkanten (16) der Tropfschale senkrechte nach unten gerichtete Randleisten (24) aufweisen, in denen die Ausnehmungen (23) ausgebildet sind, die jede einen von oben nach unten in der Einschubrichtung der Tropfschale (6) in den Ofenraum (2) gerichteten Teil aufweisen, der die eine (21) der Gleitflächen bildet.

4. Kombinierter elektrischer Ofen nach Anspruch 3, **dadurch** **gekennzeichnet,** daß die Tropfschale (6) außerdem Füße (27) aufweist, die unter den in den senkrechten Randleisten (24) der Tropfschale (6) ausgebildeten Ausnehmungen (28) so angeordnet sind, daß in der zurückgezogenen Stellung jeder Anschlag (22) zwischen einem Fuß (27) und einer Ausnehmung (28) eingeklemmt ist, wobei die Ausnehmungen (28) eine solche Tiefe bezüglich des Bodens (4) haben, daß in der zurückgezogenen Stellung der Boden (31) der Tropfschale (6) auf der Eingangsleiste (32) des Ofenraums (2) ruht und so das Zurückziehen und Kippen der Tropfschale (6) begrenzt.

5. Kombinierter elektrischer Ofen nach einem der vorangehenden Ansprüche, **dadurch gekennzeichnet,** daß die Stützvorrichtung (7) des Spießes (8) aus zwei gegenüberliegenden Haltern (11, 12, 13) besteht, die jeder einerseits gegenüber der Tropfschale (6) durch eine isolierende Basis (14), die in in der Seitenkante (16) der Tropfschale ausgebildeten Öffnungen (15) montiert ist, und andererseits gegenüber dem Spieß (8) durch eine isolierende Ringscheibe (17, 18), die vom Spieß (8) durchsetzt ist, isoliert sind.

6. Kombinierter elektrischer Ofen nach Anspruch 5, **dadurch** **gekennzeichnet,** daß der Drehantrieb (9) an der Rückwand (5) angeordnet ist und der Spieß (8) längs im Ofenraum (2) angeordnet ist und die zwei Halter (11) feststehen, so daß der Spieß (8) automatisch mit dem Drehantrieb (9) gekuppelt wird, wenn die Tropfschale (6) in die Grillstellung gebracht wird.

7. Kombinierter elektrischer Ofen nach Anspruch 5, **dadurch** **gekennzeichnet,** daß der Drehantrieb an einer Seitenwand (3) angeordnet ist und der Spieß (8) quer zum Ofenraum (2) angeordnet ist, und einer der Halter (12) mit einer Ringscheibe (17) ausgerüstet und fest an der Tropfschale (6) montiert ist, während der andere Halter (13) beweglich montiert und in Richtung auf den Drehantrieb (9) schwenkbar ist, so daß, wenn die Tropfschale (6) ihre Grillstellung einnimmt, der Spieß (8), der mit der anderen Ringscheibe (18) verschiebungsfest verbunden ist, durch Schwenken des Halters (13) mit dem Drehantrieb (9) gekuppelt wird.

8. Kombinierter elektrischer Ofen nach einem der Ansprüche 5 bis 7, **dadurch gekennzeichnet,** daß die Tropfschale (6) aus Metall ist, die Anschläge (22), die Basen (14) und die isolierenden Ringscheiben (17, 18) und der Drehantrieb (19) aus dielektrischen Materialien vom Typ Keramikmaterial hergestellt sind, während der Spieß (8) und die Halter (11, 12, 13) aus Metall sind.

## Claims

1. Combined electric oven intended to cook food by means of an electric element (1) combined with microwave heating and comprising, in a cavity (2) defined by walls (3, 4, 5) and a door, a dripping pan (6) equipped with a means of support (7) receiving a spit (8) and able to occupy two positions, either a cooking position where the dripping pan (6) is inside the cavity (2) and the spit (8) is brought into line with a rotary drive (9) mounted on one of the walls (3, 5), or a withdrawn position where the dripping pan (6) is at least partly outside the cavity (2) and the spit (8) is released from the drive (9),
characterised in that the dripping pan (6) and the walls (3) of the cavity (2) comprise means of cooperation (19) which comprise mutually sliding surfaces (20, 21) arranged in such a way that, prior to the cooking position, the surfaces (20, 21) exert a force F on each other directed towards the back (5) of the cavity (2) acting to position the dripping pan (6) automatically in its cooking position.

2. Combined electric oven according to Claim 1, characterised in that the sliding surfaces (20, 21) are carried, on the one hand, by stops (22) arranged on the lateral walls (3) of the cavity a short distance from the bottom (4) and, on the other hand, by recesses (23) made respectively in corresponding lateral edges (16) of the dripping pan (6).

3. Combined electric oven according to Claim 2, characterised in that the stops (22) consist of two cylindrical studs situated opposite each other whilst, the lateral edges (16) of the dripping pan having vertical edges (24) directed downwards, the recesses (23) are made in the said edges (24) and each have a part which slopes downwards in the direction in which the dripping pan is inserted into the cavity (2) and which constitutes one (21) of the sliding surfaces.

4. Combined electric oven according to Claim 3, characterised in that the dripping pan (6) has, in addition, lugs (27) disposed below recesses (28) made in the vertical edges (24) of the dripping pan so that, when it is in the withdrawn position, each stop (22) is wedged between a lug (27) and a recess (28), the recesses (28) having a depth in relation to the bottom (4) such that, in the withdrawn position, the bottom (31) of the dripping pan (6) rests on the front edge (3) of the cavity (2), in such a way as to limit the withdrawal and tilting of the dripping pan (6).

5. Combined electric oven according to any one of the preceding claims, characterised in that the support means (7) for the spit (8) consist of two opposed supports (11, 12, 13) which are each insulated, on the one hand, from the dripping pan (6) by an insulating base (14) mounted in openings (15) made in the edge (16) of the dripping pan and, on the other hand, from the spit (8) by an insulating ring (17, 18) through which the spit (8) passes.

6. Combined electric oven according to Claim 5, characterised in that, the drive (9) being disposed on the back wall (5) and the spit (8) being disposed longitudinally to the cavity (2), the two supports (11) are fixed, so that the spit (8) is automatically coupled with the drive (9) when the dripping pan (6) is brought into the cooking position.

7. Combined electric oven according to Claim 5, characterised in that, the drive being disposed on a lateral wall (3) and the spit (8) being disposed transversely to the cavity (2), one of the supports (12) is equipped with a ring (17) and is mounted in a fixed position on the dripping pan (6), whilst the other support (13) is mounted so as to be able to move and can tilt towards the drive (9), so that, when the dripping pan (6) occupies its cooking position, the spit (8) being fixed to the other ring (18) with respect to translational movement, the tilting of the said support (13) causes the spit (8) to be coupled with the drive (9).

8. Combined electric oven according to any one of Claims 5 to 7, characterised in that, the dripping pan (6) being made of metal, the stops (22), bases (14), insulating rings (17, 18) and drive (9) are made of dielectric materials of the ceramic type, whilst the spit (8) and the supports (11, 12, 13) are made of metal.
